# EUROPEAN PATENT APPLICATION

(11) **EP 1 897 538 A1**
(43) Date of publication of application: **12.03.2008**
(21) Application number: 06291344.7
(22) Date of filing: 23.08.2006
(51) Int. Cl.: A61K 9/16, A61K 9/51, A61K 39/36

(54) **Formulations for antigen-specific tolerance induction**

(71) Applicant: STALLERGENES SA, 92183 Antony Cedex (FR); CEVA SANTE ANIMALE, 33500 Libourne (FR)
(72) Inventor: Bardon, Thierry, 33270 Bouliac (FR); Betbeder, Didier, 59263 Houplin-Ancoisne (FR); Moingeon, Philippe, 92160 Antony (FR); Van Overtvelt, Laurence, 91300 Massy (FR); Razafindratsita, Alain, 92160 Antony (FR)
(74) Representative: Colombet, Alain André

(57) **Abstract**

The present invention relates to a synthetic particulate vector adapted for preventing and/or treating a pathological reaction of the immune system of an individual by inducing a specific tolerance towards at least one antigen involved in said pathological reaction, comprising:
- a particle comprising a non-liquid hydrophilic core, wherein the non-liquid hydrophilic core comprises a cross-linked polysaccharide; and
- said at least one antigen involved in the pathological reaction.

## Description

The invention relates to a synthetic particulate vector adapted for preventing or treating pathological immune reaction through antigen-specific tolerance induction, to immunotherapeutic compositions containing the same, or to the use of the synthetic particulate vector for preventing or treating pathological immune reactions through antigen-specific tolerance induction.

In a number of pathologies, such as allergy, graft rejection, or autoimmune disease, the immune system, through its non-specific component (i.e. inflammation) or its specific component, has a detrimental action on the organism of the individual harbouring it. Among the numerous strategies which have been devised to prevent or to treat these immune-related pathologies, one of the most promising is the induction of antigen-specific tolerance.

Antigen-specific tolerance may be defined as the absence or a reduction in intensity, of one or several immune responses, particularly the responses which are responsible for the detrimental action on the organism, to a specific antigen, in the setting of an otherwise normal immune system. Existence of an antigen-specific tolerance does not preclude that some immune reactions towards the antigen, in particular non-pathological immune reactions, be maintained. The major methods for inducing antigen-specific tolerance involve systemic administration of high doses of soluble antigen, mucosal administration of antigens, oral ingestion of antigens or intrathymic injection.

In the frame of mucosal administration, the sublingual route, for instance, has been recently explored for antigen administration in a variety of conditions, especially in the field of immunotherapy of allergy. Allergen-specific sublingual immunotherapy (SLIT) indeed represents a safe and efficient non invasive alternative to subcutaneous immunotherapy (see e.g. Bahceciler et al. (2005) Int. Arch. Allergy Immunol., 136:287-294). In this method, it is thought that the antigen is taken-up by submucosal Langerhans cells which are responsible for the allergen-specific activation of regulatory T cells which themselves induce the inhibition of helper T cells, particularly Th2 cells, thereby inducing a tolerance to this allergen. SLIT was shown to increase allergen-specific IgG4 levels with a limited impact on specific IgE responses. Such changes in the IgE/IgG4 ratio were found in some studies to correlate with a decrease in the late-phase skin reaction to the allergen and with overall clinical efficacy of the vaccine

The methods taking advantage of mucosal administration of the antigen are effective for the prophylactic or the therapeutic management of immune-related pathologies in animal models. However, there is still a need for vectors which enhance the activation of the tolerance mechanisms.

A mucoadhesive formulation based on a cross-linked polysaccharide-based (NPS) synthetic particulate vector comprising an antigen was unexpectedly found by the present Inventors to dramatically enhance antigen-specific immunotolerance in a murine model of asthma following mucosal administration. More specifically, NPS-formulated ovalbumine (OVA) was administered sublingually to Balb/c mice sensitized to ovalbumin. When compared with ovalbumin alone or untreated animals, this formulation enhanced the clinical efficacy of the vaccine as determined by improvement of the respiratory function and a decrease of inflammation of the lung (i.e. bronchial mucus secretion and specific cellular infiltration). Moreover, this mucoadhesive formulation was found to be superior in tolerance induction to a commonly used mucoadhesive biofilm (DELSTRIP).

Thus, the present invention relates to a synthetic particulate vector adapted for preventing and/or treating a pathological reaction of the immune system of an individual in particular by inducing a specific tolerance towards at least one antigen involved in said pathological reaction, comprising:
- a particle comprising a non-liquid hydrophilic core, wherein the non-liquid hydrophilic core comprises a cross-linked polysaccharide; and
- said at least one antigen involved in the pathological reaction.

The present invention also relates to an immunotherapeutic composition comprising an above-defined particulate vector, in a pharmaceutically acceptable carrier.

The present invention also relates to the use of a particulate vector as defined above, for the manufacture of a medicament intended for preventing and/or treating a pathological reaction of the immune system of an individual in particular by inducing a specific tolerance towards at least one antigen involved in said pathological reaction.

The present invention also relates to a method for preventing or treating a pathological reaction of the immune system in an individual in particular by inducing a specific tolerance towards at least one antigen involved in said pathological reaction comprising administering to said individual a prophylactically or therapeutically effective quantity of a particulate vector as defined above.

### Particle

The particles which can be used in the invention are described and can be prepared as described in particular in the following publications: WO 94/20078 or EP 0 782 851 (BVSM), WO 96/06638 (NPS, CA and BVSM) and US 6 096 291 (SMBV); the content in the brackets indicates how they are respectively referred to in these publications.

The cross-linked polysaccharide according to the invention comprises polysaccharides which may be derived from any saccharide monomer. Glucose is the preferred monosaccharide. The polymers may be formed from the monomers in either the alpha or beta orientation, and may be linked at the 1-4 or 1-6 positions of each saccharide unit. As intended herein polysaccharides encompass oligosaccharides and preferably have a molecular weight between 1,000 to 2,000,000 daltons, preferably 2,000 to 100,000 daltons, and most preferably 3,000 to 10,000 daltons.

The preferred polysaccharides comprised in the cross-linked polysaccharide according to the invention are starch (glucose alpha 1-4 polymers) and dextran (glucose alpha 1-6 polymers derived from bacteria). Starch is especially preferred. Starch from any of the well known sources of starch is suitable. Some suitable sources of starch include, for example, potato, wheat, corn, etc. Other suitable polysaccharides include, for example, pectins, amylopectins, chitosan, and glycosaminoglycan.

The polysaccharides comprised in the cross-linked polysaccharide according to the invention may also be derivatives of hydrolysates of the polysaccharides mentioned above. Some preferred hydrolysates of starch include, for example, acid hydrolyzed starch, such as dextrins, or enzyme hydrolyzed starch, such as maltodextrins. The hydrolysis degree of the polysaccharide or oligosaccharide is determined by the reducing power of the hydrolysate, commonly expressed as the Dextrose Equivalent (DE). The DE range preferably varies between 2 to 20, preferably 2 to 12.

The cross-linked polysaccharide according to the invention may be naturally or chemically cross-linked. Cross-linking processes are well-known in the art and can be carried out by means of bi- or tri-functional agents, such as epichlorohydrin or phosphorus oxychloride.

Ionic groups (preferably 0 to 3 milliequivalents, more preferably 0 to 2 milliequivalents, of ionic charge per gram) are optionally grafted to the core of cross-linked polysaccharide. The ionic groups may be anionic groups or cationic groups. The particles preferably have a minimum of 0.2, 0.4, 0.6, or 0.8 milliequivalents of ionic charge per gram of cross-linked polysaccharide core, and a maximum of 1.2, 1.4, 1.6, 1.8, 2.0 or 2.2 milliequivalents of ionic charge per gram of cross-linked polysaccharide core. Methods are known in the art for grafting ionic groups to polysaccharides.

The cross-linked polysaccharide may be made anionic by grafting a negatively charged or acidic group. Some suitable anionic groups grafted to the cross-linked polysaccharide include, for example, phosphate, sulfate, or carboxylate. Anionic groups may be grafted onto polysaccharides by treatment with an activated derivative of a polyhydric acid, such as phosphoric acid, sulfuric acid, succinic acid, or citric acid. Activated derivatives of polyhydric acids include, for example, acyl halides, anhydrides, and activated esters. A preferred anionic group is phosphate grafted via treatment with phosphorous oxychloride.

The cross-linked polysaccharide may be made cationic by grafting a positively charged or basic group. Some suitable cationic groups grafted to the cross-linked polysaccharide include, for example, quaternary ammonium ions, and primary, secondary, or tertiary amines. Other suitable groups that can be grafted to the cross-linked polysaccharide include, for example, choline, 2-hydroxypropyltrimethylammonium, 2-dimethylaminoethanol, 2-diethylaminoethanol, 2-dimethylaminoethylamine, and 2-diethylaminoethylamine. Preferably, the cationic group is quaternary ammonium (thus forming e.g. SBMV-Q). These groups may be conveniently grafted onto polysaccharides by methods known in the art, such as, for example, by contacting polysaccharides with a suitable derivative of the respective alkyl group, such as a chloride, bromide, iodide, or epoxide.

Another suitable method for grafting cationic groups onto polysaccharides includes grafting a polyhydric acid, as described above, and then using a free acid group, such as a free carboxylate group, to graft the basic group via, for example, an amide or ester bond. Amino acids are convenienly grafted this way. Some suitable examples of amino acids include, for example, glycine, alanine, glutamic acid or aspartic acid.

Optionally, the cross-linked polysaccharide core is at least partially coated with a layer of amphiphilic compounds (e.g. L-SMBV) and/or a layer of lipidic compounds.

The layer of lipid compounds may covalently coat the cross-linked polysaccharide core either partially or completely. The lipid layer preferably comprises natural fatty acids, as described in International PCT Application WO 94/23701.

The layer of amphiphilic compounds preferably adheres to the cross-linked polysaccharide, or to the optional lipid layer, by means of non-covalent bonds, such as by means of ionic or hydrogen bonds. The amphiphilic compounds suitable for the coating are selected to confer a physico-chemical environment appropriate to the substance, the mode of mucosal administration, and the desired effect.

The layer of amphiphilic compounds may comprise any amphiphilic compound that can be adsorbed on the surface of the core. Preferably, the amphiphilic coating comprises mainly a natural or synthetic phospholipid or ceramide, or a mixture thereof.

The phosphate group of the phospholipid may optionally be grafted to ionic or neutral groups. Some suitable phospholipids include, for example, phosphatidyl choline, phosphatidyl hydroxycholine, phosphatidyl ethanolamine, phosphatidyl serine, and phosphatidyl glycerol. A preferred phospholipid is dipalmitoyl phosphatidylcholine (DPPC).

The layer of amphiphilic compounds may also comprise a derivative of a phospholipid or ceramide. Some suitable derivatives of phospholipids include PEG- phospholipids, and phospholipids grafted to other molecules or polymers.

The layer of amphiphilic compounds may also comprise other amphiphilic compounds, either by themselves or in combination with the phospholipids, ceramides, or derivatives described above. Some suitable examples of such other amphiphilic compounds include poloxamers, modified polyoxyethylene, and other detergents and surface active compounds.

Additional compounds and mixtures thereof may be added to the phospholipids or ceramides in the amphiphilic coating. Some examples of such additional compounds include fatty acids, steroids (such as cholesterol), triglycerides, lipoproteins, glycolipids, vitamins, detergents, and surface active agents.

The preparation may normally be conveniently carried out, either as a simple one-step process (in case of an uncoated core) or as a two step process: the core is first prepared, optionally coated by a lipidic layer, and then coated with an amphiphilic layer.

The diameter of the particle may be comprised between 10 nm and 5 µm and preferably between 20 and 200 nm.

### Antigen

As intended herein an antigen involved in a pathological reaction of the immune system relates to a compound which is liable to induce a reaction of the immune system directed against it and which is responsible for the onset or the maintaining of an immune reaction which is targeted against tissues or cells of the organism which harbours said immune system.

The antigen may of any type. In particular, it can be a protein, a polypeptide or a peptide, a carbohydrate, a lipid, a nucleic acid, such as DNA or RNA, or a virus, in particular a recombinant virus.

The antigen may be selected from the group consisting of an allergen, an auto-antigen and a graft-specific antigen.

In a preferred embodiment, the antigen is an allergen. An "allergen" is defined as a substance, usually a protein, which elicits the production of IgE antibodies in predisposed individuals. Similar definitions are presented in the following references: Clin. Exp. Allergy, No. 26, pp. 494-516 (1996); Mol. Biol. of Allergy and Immunology, ed. R. Bush, Immunology and Allergy Clinics of North American Series (August 1996).

Preferably the antigen is a protein allergen, which means any amino acid chain likely to trigger an allergic response, including short peptides of about 6 to 20 aminoacids, polypeptides, or full proteins.

Non limitative examples of allergens include pollen allergens (tree-, herb, weed-, and grass pollen allergens), insect allergens (inhalant, saliva and venom allergens, e.g. mite allergens, cockroach and midges allergens, hymenopthera venom allergens), animal hair and dandruff allergens (from e.g. dog, cat, horse, rat, mouse etc.), and food allergens.

For instance, the protein allergen may be selected from the group consisting of a protein allergen of the genus Dermatophagoides; a protein allergen of the genus Felis; a protein allergen of the genus Ambrosia; a protein allergen of the genus Lolium; a protein allergen of the genus Cryptomeria; a protein allergen of the genus Alternaria; a protein allergen of the genus Alder; a protein allergen of the genus Betula; a protein allergen of the genus of Blomia; a protein allergen of the genus Quercus; a protein allergen of the genus Olea; a protein allergen of the genus Artemisia; a protein allergen of the genus Plantago; a protein allergen of the genus Parietaria; a protein allergen of the genus Canine; a protein allergen of the genus Blattella; a protein allergen of the genus Apis; a protein allergen of the genus Cupressus; a protein allergen of the genus Thuya; a protein allergen of the genus Chamaecyparis; a protein allergen of the genus Periplaneta; a protein allergen of the genus Agropyron; a protein allergen of the genus Secale; a protein allergen of the genus Triticum; a protein allergen of the genus Cynorhodon ; a protein allergen of the genus Juniperus ; a protein allergen of the genus Dactylis; a protein allergen of the genus Festuca; a protein allergen of the genus Poa; a protein allergen of the genus Avena; a protein allergen of the genus Holcus; a protein allergen of the genus Anthoxanthum; a protein allergen of the genus Arrhenatherum; a protein allergen of the genus Agrostis; a protein allergen of the genus Phleum; a protein allergen of the genus Phalaris; a protein allergen of the genus Paspalum; and a protein allergen of the genus Sorghum.

Examples of various known protein allergens derived from some of the above-identified genus include : Betula (verrucosa) Bet v I ; Bet v II ; Blomia Blo t I; Blo t III; Blo t V; Blo t XII; Cynorhodon Cyn d I; Dermatophagoides (pteronyssinus or farinae) Der p I; Der p II; Der p III; Der p VII; Der f I; Der f II; Der f III; Der f VII; Felis (domesticus) Fel d I; Ambrosia (artemiisfolia) Amb a I.1; Amb a 1.2; Amb a 1.3; Amb a I.4; Amb a II; Lollium (perenne) Lol p I; Lot p II; Lol p III; Lot p IV; Lol p IX (Lol p V or Lol p Ib); Cryptomeria (japonica) Cry j I; Cry j II; Can f I; Can f II; Juniperus (sabinoides or virginiana) Jun s I; Jun v I; Juniperus (ashei) Jun a I; Jun a II; Dactylis (glomerata) Dac g I; Dac g V; Poa (pretensis) Poa p I; PhI p I; PhI p V; PhI p VI and Sorghum (halepensis) Sor h I.

Food allergens may originate from milk and milk products, eggs, legumes (peanuts and soy), tree nuts, wheat, crustaceans, fish, and mollusks. In particular, food allergens may be ovalbumine or gluten.

In addition, similar to allergy, autoimmune diseases such as Type I diabetes, multiple sclerosis, and rheumatoid arthritis are generally accepted as being the result of an antigen specific T cell mediated response against an antigen which in the case of autoimmune disease is an auto-antigen, i.e. an antigen that belongs to the body's own tissue. The same applies to the phenomenon of graft rejection, where the antigen belongs to the graft tissue, possibly coming from another individual or even from another animal species.

In another embodiment, the antigen is involved in an autoimmune disease or graft rejection.

A number of antigens (i.e. auto-antigens) have been found to cause symptoms in autoimmune diseases (i.e. auto-antigens such as insulin; myelin basic protein; rh factor; acetylcholine receptors; thyroid cell receptors; basement membrane proteins; thyroid proteins; ICA-69 (PM-1); glutamic acid decarboxylase (64K or 65K); proteolipid protein (PLP), myelin associated glycoprotein (MAG), Collagen (Type II), Heat Shock Protein and carboxypeptidase H) such as diabetes, rheumatoid arthritis, and multiple sclerosis.

Furthermore, graft-specific antigen may elicit a graft versus host disease which may lead ultimately to rejection of the graft.

### Synthetic particulate vector

In the synthetic particulate vector according to the invention, the antigen and the particle are associated together, preferably through non-covalent interactions.

There is flexibility in where the one, or more than one, antigen is located in the synthetic particulate vector. For example, the one, or more than one kind of antigen may be located in the inner core of the cross-linked polysaccharide. Alternatively, the one, or more than one, substance may be located at the outer surface of the cross-linked polysaccharide.

If the cross-linked polysaccharide is coated with an amphiphilic layer and/or a lipid layer, the one, or more than one, antigen may be located in the inner core of the amphiphilic compound and/or lipid layer. Alternatively, the one, or more than one, substance may be located at the outer surface of the amphiphilic compound layer and/or lipid layer.

If more than one antigen per synthetic particulate vector is administered to a mammal, some or all of the antigens may be located in the same part of the synthetic particulate vector. Alternatively, some or all of the substances may be located in the different parts of the synthetic particulate vector.

Methods are known for directing substances to various parts of synthetic particulate vector. See International PCT Application WO094/20078 for instance.

In the synthetic particulate vector of the invention, the ratio of the dose of antigen to the dose of particle preferably ranges from 1:1 to 1:100 (weight of antigen / weight of particle).

### Immunotherapeutic compositions, medicaments and methods for preventing or treating a pathological reaction of the immune system in an antigen-specific manner

As intended herein a "pathological reaction of the immune system of an individual" relates to an immune reaction which is targeted against tissues or cells of the organism which harbours said immune system.

Such a pathological reaction is in particular selected from the group consisting of allergy, such as asthma, autoimmune disease or graft rejection.

In the context of the invention allergy relates to asthma or to the allergies due to the above-defined allergens.

In the context of the invention an autoimmune disease in particular relates to Type I diabetes, multiple sclerosis, rheumatoid arthritis, and to the diseases due to the above-defined auto-antigens.

As intended herein the term "immunotherapeutic" relates to the capacity of a substance to prevent or to treat a pathological reaction of the immune system.

In the context of the invention, the terms "to treat", "treating" or "treatment", means reversing, alleviating, or inhibiting the course of a pathological reaction of the immune system or one or more symptoms thereof.

In the context of the invention, the terms "to prevent" or "preventing", means the onset of a pathological reaction of the immune system or one or more symptoms thereof.

As used herein, the term "individual" preferably denotes a mammal, such as a rodent, a feline, a canine, and a primate. Preferably, an individual according to the invention is a human.

"Pharmaceutically" or "pharmaceutically acceptable" refer to molecular entities and compositions that do not produce an adverse, allergic or other untoward reaction when administered to an animal, or a human, as appropriate.

As used herein, "pharmaceutically acceptable carrier" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the synthetic particulate vector according to the invention, its use in the immunotherapeutic compositions, in the medicaments, or for implementing the methods for preventing or treating a pathological reaction of the immune system in an individual according to the invention is contemplated. Supplementary active ingredients can also be incorporated into the compositions.

It is thought that the particular immunotherapeutic properties of the synthetic particulate vector of the invention, unexpectedly and particularly, favour the contacting of the antigens it harbours with mucosal, in particular oromucosal, more particularly sublingual cells, such as Langerhans cells, thereby favouring antigen-specific tolerance induction.

Advantageously, the synthetic particulate vector according to the invention is mucoadhesive. Mucoadhesivity can account for the immunotherapeutic properties of the synthetic particulate vector by enabling a close and prolonged contact with a mucosa, in particular a mucosa of the oral cavity, and more particularly the sublingual mucosa, thereby enhancing-antigen specific tolerance induction.

Preferably, the synthetic particulate vector, the immunotherapeutic composition, or the medicament is to be administered by the systemic or the mucosal route, more preferably by the oromucosal route, and most preferably by the sublingual route. As such the immunotherapeutic composition and the medicament are preferably formulated in a way adapted for such administration routes.

Mucosal administration denotes any administration method, wherein the formulation in part or in full comes into contact with a mucosa. Mucosa refers to the epithelial tissue that lines the internal cavities of the body. The mucosal surface may be selected from the group consisting of a nasal, buccal, oral, vaginal, ocular, auditory, pulmonary tract, urethral, digestive tract, and rectal surface.

Oromucosal administration comprises any administration method, wherein the formulation in part or in full comes into contact with the mucosa of the oral cavity and/or the pharynx of the patient.

Oromucosal administration includes in particular sublingual, perlingual (i.e. through the tongue mucosa) and oral administrations.

The synthetic particulate vector, the immunotherapeutic compositions or the medicaments according to the invention can be administered in various forms, such as dispersed forms, e.g. in suspensions or gels, or as dry forms, e.g. in powders, tablets, capsules, lyoc, or forms suitable to be administered in a metered-dosing device,

In the frame of methods for preventing or treating pathological reactions of the immune system, the immunotherapeutic compositions or the medicaments, according to the invention, the antigen may be pre-loaded to the particle prior to administration to the individual. Alternatively, the antigen and the particle may be mixed together at the time of administration to the individual.

In the frame of methods for preventing or treating pathological reactions of the immune system, the immunotherapeutic compositions or the medicaments, according to the invention, any conventional or exploratory, synthetic or biological adjuvant for vaccination, including heat-labile enterotoxin (LT), cholera-toxin (CT), cholera toxin B subunit (CTB), polymerised liposomes, mutant toxins, probiotic bacteria, oligonucleotides, RNA, siRNA, DNA, lipids can be associated to the synthetic particulate vectors according to the invention to enhance tolerance induction. For oromucosal administration, the adjuvants may be preferably a bacterium selected from a *Bifidobacterium* and a lactic acid bacterium, or a combination of a corticosteroid with vitamin D3 or any metabolite or analog of the latter.

In the frame of methods for preventing or treating pathological reactions of the immune system, the immunotherapeutic compositions or the medicaments, according to the invention, the administration regimen may be repeated for a period of less than 6 weeks to up to three years.

Furthermore prevention or treatment may be effected with a plurality of different antigens. This may be achieved either with one type of synthetic particulate vector containing a plurality of antigens or with a plurality of synthetic particulate vectors containing one or more antigens each.

Preferably, in the frame of methods for preventing or treating pathological reactions of the immune system, the immunotherapeutic compositions or the medicaments, according to the invention, the range of dose for the synthetic particulate vector according to the invention may be between 10 µg to 100 mg.

The invention will be further illustrated in view of the following figures and examples.

### FIGURES

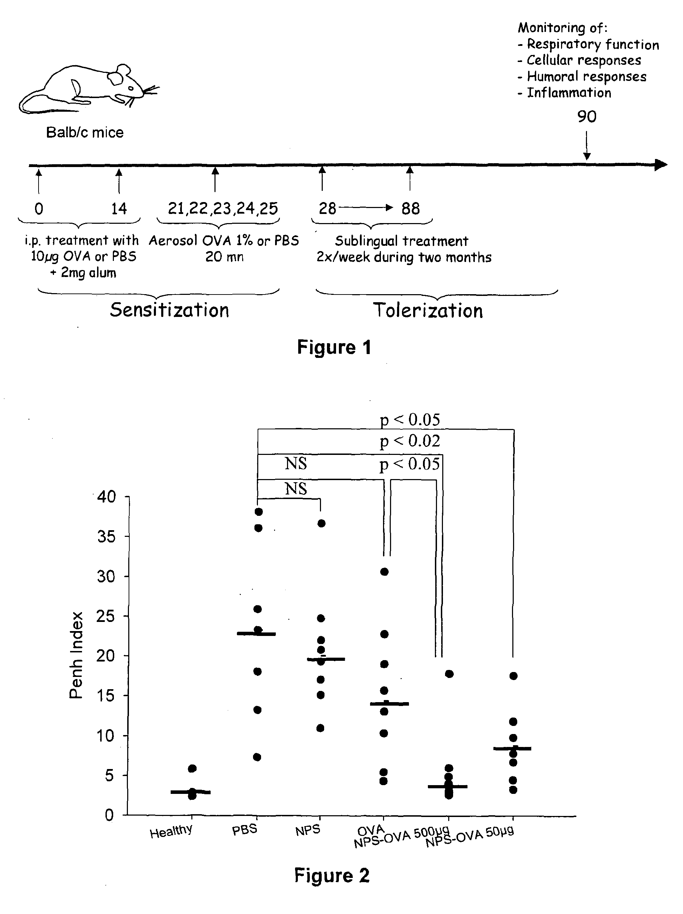

Figure 1: Experimental design. The effect of NPS-formulated OVA or Delstrip-formulated OVA was assessed in a murine model of ovalbumin-induced asthma. First, animals were sensitized by two intraperitoneal (i.p.) injections at 14 days interval with 10µg ovalbumin (OVA) adsorbed on 2 mg Al(OH)₃ or phosphate buffer saline (PBS). This priming was followed by a challenge consisting of a 20 min aerosol with 1% w/v OVA for 5 consecutive days. Secondly, animals were desensitized through the sublingual route using PBS, 500 µg of OVA, NPS alone, Delstrip alone, NPS formulated OVA (500 or 50 µg) or Delstrip formulated OVA (500 µg), 2 times per week during 2 months. Measurements of airway responsiveness were performed by whole body plethysmography. After sacrifice of mice, spleen and lung were removed for in vitro cytokine assays, inflammation analysis (cellular infiltration and mucus production) and antibody levels determination.
Figure 2: Airway responsiveness after tolerization with NPS formulated OVA. The airway responsiveness was determined for healthy mice (group healthy), mice sensitized with OVA and tolerized with PBS (group PBS), mice sensitized with OVA and tolerized with OVA (group OVA), mice sensitized with OVA and tolerized with NPS alone (group NPS) and mice sensitized with OVA and tolerized with NPS formulated OVA 500 µg or 50µg (group NPS-OVA 500µg or group NPS-OVA 50µg) by measurement of Penh value in response to methacholine (50 mg/ml). Four to eight mice per group were displayed (horizontal bar = median).

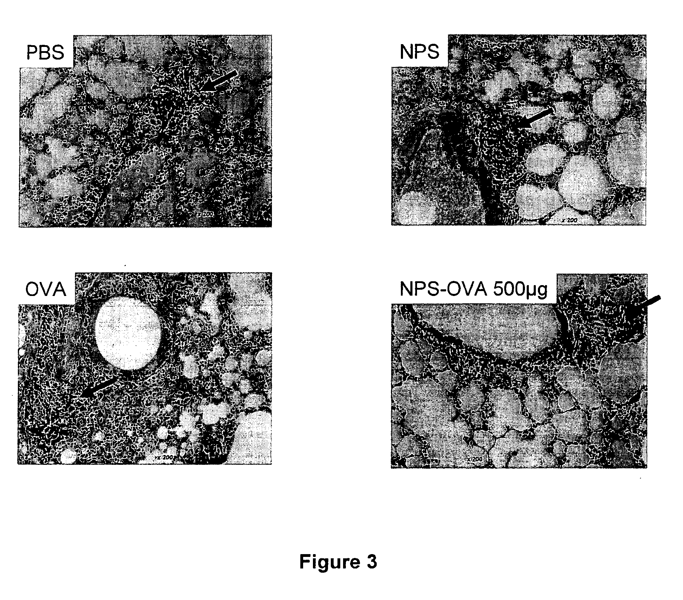

Figure 3: Inflammation of Lung after tolerization with NPS formulated OVA. Representative lung sections obtained after tolerization with PBS, NPS alone, OVA or NPS formulated OVA 500 µg. Paraffin tissue sections were stained with HES (Hematoxylin, eosin, safran). Arrow indicates specific cellular infiltration.

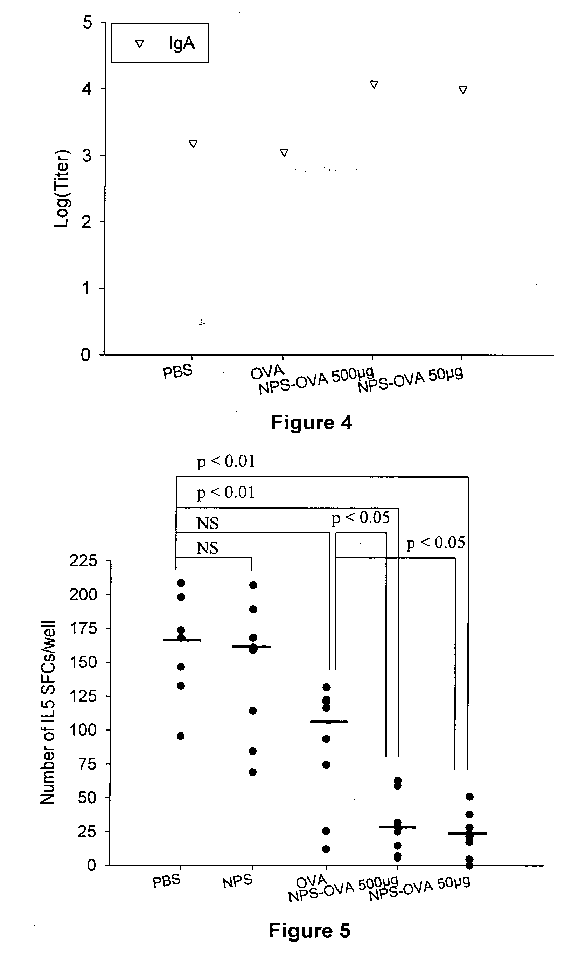

Figure 4: Mucosal response after tolerization with NPS formulated OVA. OVA-specific IgA antibody level was measured in lung of mice tolerizated with PBS, OVA, NPS formulated OVA 500µg or NPS formulated OVA 50µg.
Figure 5: IL-5 production after tolerization with NPS formulated OVA. IL-5 levels in spleen cells of mice tolerizated with PBS, OVA, NPS, NPS formulated OVA 500µg or NPS formulated OVA 50µg were measured by Elispot.

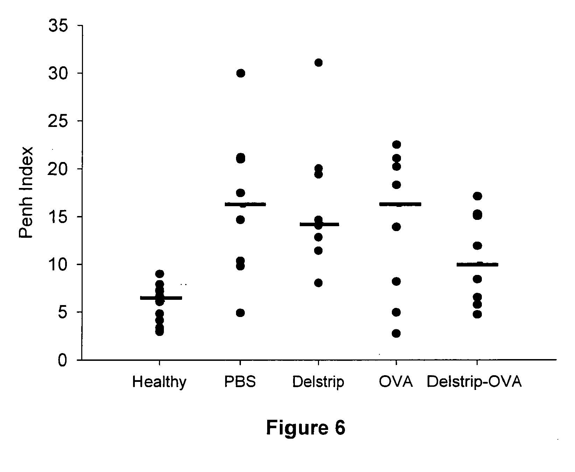

Figure 6: Airway responsiveness after tolerization with Delstrip formulated OVA. The airway responsiveness was determined for healthy mice (group healthy), mice sensitized with OVA and tolerized with PBS (group PBS), mice sensitized with OVA and tolerized with OVA (group OVA), mice sensitized with OVA and tolerized with Delstrip alone (group Delstrip) and mice sensitized with OVA and tolerized with Delstrip formulated OVA 500 µg (group Delstrip-OVA) by measurement of Penh value in response to methacholine (50 mg/ml). Eight mice per group were displayed (horizontal bar = median).

### EXAMPLE

### Materials and methods

### Animals, culture medium, reagents and antigens

Six-week-old female BALB/c mice were purchased from Charles River (L'Arbresle, France) and maintained on an ovalbumine (OVA)-free diet. Each experimental group consisted of four to eight mice. The international levels of ethical standards for the use of animals were applied.

Ovalbumin (OVA, Garde V) was purchased from Sigma Chemicals. Phosphate-buffer saline (PBS) and alum were purchased from Invitrogen (France) and Pierce (Rockford, Illinois), respectively.

### Mucoadhesive formulation

NPS: Cationic particles of 60 nm were prepared with maltodextrins reticulated with epichlorohydrin and derivatized using cationic ligands (GTMA). The nanoparticles were loaded with ovalbumin using 1/3.5 (weight protein/weight nanoparticle) ratio.
Delstrip: A drop of 5 µl of OVA (100mg/ml) was applied on a thin cellulose film (3 mm) from Cardinal health

### Mice sensitization and tolerization

Sensitization was performed by two intraperitoneal (i.p.) injections at 14 days interval with 10 µg OVA adsorbed on 2 mg Al(OH)₃ in a volume of 100 µl. This priming was followed by a 20 min aerosol challenge with 1% w/v OVA on 5 consecutive days using an aerosol delivery system (Buxco Europe; Ltd, UK). Control animals were sham treated with sterile PBS.

For tolerance induction in a therapeutic model, sensitized mice were sublingually treated with OVA (500 µg), NPS-OVA (500 µg), NPS-OVA (50 µg) or Delstrip-OVA (500 µg), two times per week during 2 month in a final volume of 20 µl. Control mice were OVA-sensitized animals receiving sham tolerization with PBS or NPS or Delstrip formulation alone.

### Determination of airway responsiveness

Measurements of airway responsiveness were performed by whole body plethysmography (Buxco Europe; Ltd, UK). Airflow signals were recorded in response to aerosolized methacholine (50 mg/ml), 1 minute, four times. The higher value of Penh (enhanced pause) was taken account for the calculation of the Penh index (Penh index = Penh measured/Penh PBS).

### Lung histology

Mice were killed by cervical dislocation. Lung were isolated and embedded in paraffin. Micrometer paraffin sections were stained with HES (Hematoxylin, eosin, safran).

### Analysis of cellular immune responses by Elispot

Mice were killed by cervical dislocation. Spleen cells were isolated and plated in triplicate in 96-well flat-bottomed ELISPOT plated (Millipore, Massachussets) at densities of 2x10⁵ cells per well. Different stimulation conditions were used: medium alone, OVA (100µg/ml) and PMA (50ng/ml, Sigma)/ionomycine (500ng/ml, Sigma) which was used as positive control. Spot forming cells (SFCs) were monitored for IL-5 and assessed with an Elispot reader (Carl Zeiss Microimaging, GmbH). Elispot kit was from R&D systems Europe.

### Determination of lung allergen-specific antibody levels

One lobe of lung was teased in a complete medium and cell suspensions were filtered through 297 µm (mesh sieve, Sigma Aldrich). The recovered cell suspensions were centrifuged at 200g for 5 min at room temperature. The supernatant was collected for IgA level determination by Elisa. Briefly, microplates were coated with a goat anti-mouse IgA (0.1 µg/well) and different dilutions of supernatants were incubated for 1 hours at 37°C. OVA-digoxigenin (dilution 1/20, Roche, France) plus HRPO-conjugated rabbit anti-digoxigenin (dilution 1/1000, Roche, France) were added and incubated for 1 hour at 37°C. ABTS substrate (Roche, France) was used and O.D. read at 405 nm with a microplate reader.

### Statistical analysis

Statistical significance was determined with Student's t-test and ANOVA for comparing group samples. A p value below 0.05 was considered statistically significant.

### Results

The inventors have tested the capacity of a mucoadhesive formulation (NPS or Delstrip) to induce tolerance by the sublingual route (Figure 1).

OVA-sensitized mice exhibit a high airway responsiveness (Figure 2) and severe bronchial inflammation (Figure 3), thereby validating the asthma model.

Sublingual treatment with NPS-formulated OVA (tolerization) enhances dramatically the efficacy of OVA specific immunotherapy in mice, as shown by a normalization of the respiratory function (Figure 2) and reduction of bronchial inflammation (Figure 3), even at low doses of OVA (50 µg).

Sublingual treatment with NPS formulated OVA led to increase of OVA-specific IgA secretion in the lung (Figure 4).

The number of IL-5 spot forming cells was reduced in spleen cell cultures of mice treated with NPS formulated OVA compared to PBS-treated mice (or animals treated with OVA alone) (Figure 5).

Finally it is to be noted that not all mucoadhesives are as efficient, since whereas NPS was particularly efficient, the DELSTRIP biofilm was only slightly more efficient than soluble OVA (Figure 6).

## Claims

1. A synthetic particulate vector adapted for preventing and/or treating a pathological reaction of the immune system of an individual by inducing a specific tolerance towards at least one antigen involved in said pathological reaction, comprising:
- a particle comprising a non-liquid hydrophilic core, wherein the non-liquid hydrophilic core comprises a cross-linked polysaccharide; and
- said at least one antigen involved in the pathological reaction.

2. The synthetic particulate vector according to claim 1, wherein the diameter of the particle is from 10 nm to 5 µm.

3. The synthetic particulate vector according to claim 1 or 2, wherein the diameter of the particle is from 20 nm to 200 nm.

4. The synthetic particulate vector according to any of claims 1 to 3, wherein the polysaccharide is cross-linked by means of bi- or tri-functional agents.

5. The synthetic particulate vector according to any of claims 1 to 4, wherein the polysaccharide is selected from the group consisting of starch, dextran, dextrin, and maltodextrin.

6. The synthetic particulate vector according to any of claims 1 to 5, wherein the core is grafted by ionic groups.

7. The synthetic particulate vector according to any of claims 1 to 6, wherein the core is at least partially coated with a layer of amphiphilic compounds and/or a layer of lipidic compounds.

8. The synthetic particulate vector according to any of claims 1 to 7, wherein the antigen is selected from the group consisting of an allergen, an auto-antigen and a graft-specific antigen.

9. The synthetic particulate vector according to any of claims 1 to 8, wherein the antigen is an allergen.

10. The synthetic particulate vector according to claim 9, wherein said allergen is selected from the group consisting of pollen allergens, insect allergens, animal hair and dandruff allergens, and food allergens.

11. An immunotherapeutic composition comprising a particulate vector as defined in any of claims 1 to 10, in a pharmaceutically acceptable carrier.

12. An immunotherapeutic composition according to claim 11, in the form of a suspension, a gel, a powder, a tablet, a capsule, or a lyoc.

13. The use of a particulate vector as defined in any of claims 1 to 10 or of an immunotherapeutic composition according to claim 11 or 12, for the manufacture of a medicament intended for preventing and/or treating a pathological reaction of the immune system of an individual by inducing a specific tolerance towards at least one antigen involved in said pathological reaction.

14. The use according to claim 13, wherein the pathological reaction is selected from allergy, autoimmune disease or graft rejection.

15. The use according to claim 13 or 14, wherein the antigen is added to the particle prior to administration to a subject.

16. The use according to claim 13 or 14, wherein the antigen and the particle are mixed together at the time of administration to a subject.

17. The use according to any of claims 13 to 16, wherein the medicament is to be administered, by the systemic or the mucosal route.

18. The use according to any of claims 13 to 17, wherein the medicament is to be administered via the oromucosal route.

19. The use according to any of claims 13 to 18, wherein the medicament is to be administered via the sublingual route.
